# EUROPEAN PATENT APPLICATION

(11) **EP 4 706 384 A1**
(43) Date of publication of application: **11.03.2026**
(21) Application number: 25200191.2
(22) Date of filing: 04.09.2025
(51) Int. Cl.: A01K 11/00, A61B 5/01, G01K 13/20

(54) **INJECTION MOLDED TYPE BIOMETRIC MONITORING EAR TAG**

(30) Priority: 09.09.2024 US 202463692286 P
(71) Applicant: Huang, Cheng Pin, New Taipei City 241523 (TW)
(72) Inventor: Huang, Cheng Pin, New Taipei City 241523 (TW)
(74) Representative: Viering, Jentschura & Partner mbB Patent- und Rechtsanwälte

(57) **Abstract**

An injection molded type biometric monitoring ear tag (100) for attaching to an ear (201) of an animal (200) to be monitored, comprising a male tag (1) and a female tag (2, 2a). The male tag (1) is disposed on a first side (202) of the ear (201) and includes a base (11) and a protrusion (12) extending from the base (11). The female tag (2, 2a) is disposed on a second side (203) of the ear (201) and includes a fixing bracket (21), a heat-conducting element (22, 22a, 22b, 22c), an electronic component (23), and an injection molded shell (24, 24a). The heat-conducting element (22, 22a, 22b, 22c) is fixed to the fixing bracket (21) and closely abuts the ear (201). The electronic component (23) is fixed to the fixing bracket (21) and coupled to the heat-conducting element (22, 22a, 22b, 22c) to sense an ear temperature value. The injection molded shell (24, 24a) encapsulates the fixed fixing bracket (21), the heat-conducting element (22, 22a, 22b, 22c), and the electronic component (23), so that the heat-conducting element (22, 22a, 22b, 22c) is exposed from the injection molded shell (24, 24a). Therefore, it forms a plug-in fixing channel (C3, C3a), thereby fixing the male tag (1) and the female tag (2, 2a) relative to each other.

## Description

This utility application claims priority to U.S. Provisional Application Serial Number 63/692,286, filed on September 9, 2024, the subject matter of which is incorporated herein by reference.

### BACKGROUND OF THE INVENTION

### (1) Field of the Invention

The invention relates to a biometric monitoring ear tag, and more particularly to an injection molded type biometric monitoring ear tag.

### (2) Description of the Prior Art

In the modem livestock industry, accurately monitoring the health of economic animals (such as pigs, cattle, sheep, etc.) is a crucial core management goal. To accurately monitor the health of economic animals, ear tag technology has been widely used in the management of economic animals. By attaching the ear tags to the ears, it not only enables individual identification but also continuously monitors important biological parameters (such as body temperature).

Traditional ear tags usually include a male tag and a female tag. The fixing method is to insert the protrusion of the male tag through the ear and then fix it into the plug-in hole of the female tag, so that the male tag and the female tag are fixed relative to each other.

In the prior arts, most female tags used for biometric monitoring are two-piece female tags, that is, an upper shell and a lower shell are used to enclose the internal electronic components. However, the two-piece female tag has the following significant disadvantages: First, in terms of production and manufacturing, not only does it require investment in developing two sets of molds, but it also requires time-consuming assembly processes afterwards. Second, after the assembly is completed, there is inevitably an assembly gap between the upper shell and the lower shell, resulting in poor waterproof, dustproof and even shockproof performance, thereby increasing the risk of damage to internal electronic components and unstable power supply.

### SUMMARY OF THE INVENTION

In view of the fact that in the prior arts, the two-piece female tag is not only costly and time-consuming to assemble, but also suffers from poor waterproof, dustproof, and even shockproof performance due to the assembly gap between the upper shell and lower shell, thereby increasing the risk of damage to internal electronic components.

Therefore, the main purpose of the present invention is to provide an injection molded type biometric monitoring ear tag, which is formed after fixing the heat-conducting element and the electronic component on the fixing bracket, and is coated with an injection molded shell to form a one-piece female tag. It not only solves the problems of high costs and time-consuming assembly and increases the feasibility of large-scale production, but also significantly improves waterproof, dustproof and even shockproof performance by eliminating assembly gaps to reduce the risk of damage to internal electronic components.

Accordingly, the necessary technical means adopted by the present invention to solve the problems of prior arts is to provide an injection molded type biometric monitoring ear tag for attaching to an ear of an animal to be monitored. The ear includes a first side and a second side. The injection molded type biometric monitoring ear tag includes a male tag and a female tag.

The male tag is disposed on the first side and includes a base and a protrusion. The base has a base contact surface facing the ear. The protrusion extends from the base contact surface, for extending from the first side through the ear to the second side.

The female tag is disposed on the second side and includes a fixing bracket, at least one heat-conducting element, an electronic component, and an injection molded shell. The fixing bracket has a bracket channel surrounded by an inner wall. The heat-conducting element is fixed to one side of the fixing bracket facing the ear and used to closely abut the ear to conduct an ear heat energy of the ear when the male tag and the female tag are fixed relative to each other. The electronic component is fixed to the fixing bracket and coupled to the heat-conducting element to sense an ear temperature value according to the ear heat energy.

The injection molded shell encapsulates the fixed fixing bracket, the heat-conducting element, and the electronic component after applying an injection molding process, so that part of the heat-conducting element is exposed on one side of the injection molded shell facing the ear. Then, it forms a plug-in fixing channel after encapsulating the inner wall, thereby the protrusion can be plugged and fixed to make the male tag and the female tag relative fixed to each other.

Among the ancillary technical means derived from the above necessary technical means, preferably, the fixing bracket includes a lower bracket and an upper bracket. The lower bracket includes a lower bracket body and two lower bracket extensions. The lower bracket body has the bracket channel. The lower bracket extensions extend from the lower bracket body respectively, and each has a snap-fit column respectively.

Following the above mentioned, the upper bracket includes an upper bracket body and two upper bracket extensions. The upper bracket body has a fitting hole. The fitting hole is used to fit the lower bracket body. The upper bracket extensions extend from the upper bracket body respectively, and each has a snap-fit hole respectively. The snap-fit holes are used to fit the snap-fit columns. The upper bracket is engaged and fixed to the lower bracket when the fitting hole fits the lower bracket body and the snap-fit holes fit the snap-fit columns.

Based on the above-mentioned auxiliary technical means, the following auxiliary technical means can be derived. Preferably, the lower bracket has at least two plug-in columns and the heat-conducting element includes a heat-conducting protrusion and two fixed ends. The fixed ends extend from the heat-conducting protrusion respectively, and each has a plug-in hole respectively. The plug-in holes are used to fit the plug-in columns, thereby the heat-conducting element is fixed to the lower bracket.

Based on the above-mentioned auxiliary technical means, the following auxiliary technical means can be derived. Preferably, the electronic component includes a circuit board. The circuit board has a limiting hole and two limiting slots. The limiting hole is used to fit the lower bracket body, and the limiting slots are used to accommodate the snap-fit columns. The circuit board is restricted and fixed between the lower bracket and the upper bracket when the limiting hole fits the lower bracket body and the limiting slots accommodate the snap-fit columns.

Based on the above-mentioned auxiliary technical means, the following auxiliary technical means can be derived. Preferably, the electronic component further includes a control module, a temperature sensing module, and a power supply module. The control module is disposed on the circuit board and has a signal processing unit. The temperature sensing module is disposed on the circuit board, electrically connected to the control module, and coupled to the heat-conducting element to sense the ear temperature value. The power supply module is disposed on the circuit board and electrically connected to the control module.

Based on the above-mentioned auxiliary technical means, the following auxiliary technical means can be derived. Preferably, the upper bracket further includes a limiting portion. The limiting portion extends from the upper bracket body facing the power supply module and is used to abut the power supply module disposed on the circuit board, thereby the power supply module is limited.

Based on the above-mentioned auxiliary technical means, the following auxiliary technical means can be derived. Preferably, the injection molded shell is a translucent shell, and the electronic component further includes at least one light-emitting module. The light-emitting module is disposed on the circuit board and electrically connected to the control module, thereby the light-emitting module is controlled to generate light transmitting the injection molded shell. More preferably, the upper bracket further includes at least one light guide portion. The light guide portion extends from the upper bracket body toward the light-emitting module, covers the light-emitting module, and includes a channel portion and an emitting portion. The channel portion has a light guide channel, and the emitting portion is connected to one side of the channel portion away from the light-emitting module, thereby the light generated by the light-emitting module is guided by the light guide channel, and then penetrates the emitting portion and emits out of the injection molded shell.

Based on the above-mentioned auxiliary technical means, the following auxiliary technical means can be derived. Preferably, the upper bracket further includes an abutment portion. The abutment portion extends from the upper bracket body. The electronic component further includes an antenna, a probe, and a communication module. The antenna abuts against the abutment portion. The probe protrudes from the circuit board and is electrically connected to the antenna. The communication module is disposed on the circuit board and electrically connected to the control module and the probe respectively, thereby the ear temperature value is controlled to be transmitted via the antenna.

Based on the above-mentioned auxiliary technical means, the following auxiliary technical means can be derived. Preferably, the electronic component further includes at least one biometric sensing module. The at least one biometric sensing module is disposed on the circuit board, electrically connected to the control module, and used to sense at least one biological parameter corresponding to the animal to be monitored. The biometric sensing module is at least one of an accelerometer, a motion and position sensor, an ultrasonic sensor, a gyroscope, a humidity sensor, an infrared sensor, a magnetometer, a heart rate sensor, an electrocardiography (ECG), a photoplethysmography sensor (PPG), an acoustic sensor, a vibration sensor, a microphone, a piezoelectric biosensor, a chemical and gas sensor, and a pressure sensor.

To summarize, the injection molded type biometric monitoring ear tag provided by the present invention is formed after fixing the heat-conducting element and the electronic component on the fixing bracket, and is coated with an injection molded shell to form a one-piece female tag. Therefore, it not only solves the problems of high costs and time-consuming assembly and increases the feasibility of large-scale production, but also significantly improves waterproof, dustproof and even shockproof performance by eliminating assembly gaps to reduce the risk of damage to internal electronic components.

The specific embodiments used in the present invention will be further explained through the following embodiments and drawings.

### BRIEF DESCRIPTION OF THE DRAWINGS

The present invention will now be specified with reference to its preferred embodiment illustrated in the drawings, in which:
FIG. 1 is a perspective view showing the female tag of the injection molded type biometric monitoring ear tag provided by the first embodiment of the present invention disposed on the second side of the ear;
FIG. 2 is a perspective view showing the male tag of the injection molded type biometric monitoring ear tag provided by the first embodiment of the present invention disposed on the first side of the ear;
FIG. 3 is a perspective exploded view of the injection molded type biometric monitoring ear tag provided by the first embodiment of the present invention;
FIG. 4 is a schematic diagram showing a perspective exploded view of the injection molded type biometric monitoring ear tag provided by the first embodiment of the present invention from another perspective;
FIG. 5 is a perspective exploded view of the female tag of the injection molded type biometric monitoring ear tag provided by the first embodiment of the present invention;
FIG. 6 is a schematic diagram showing a perspective exploded view of the female tag of the injection molded type biometric monitoring ear tag provided by the first embodiment of the present invention from another perspective;
FIG. 7 is a perspective exploded view showing the electronic component of the injection molded type biometric monitoring ear tag provided by the first embodiment of the present invention;
FIG. 8 is a perspective exploded view showing the electronic component of the injection molded type biometric monitoring ear tag provided by the first embodiment of the present invention from another perspective;
FIG. 9 is a block diagram showing the implementation system of the injection molded type biometric monitoring ear tag provided by the first embodiment of the present invention; and
FIG. 10 is a perspective exploded view of the injection molded type biometric monitoring ear tag provided by the second embodiment of the present invention.

### DESCRIPTION OF THE PREFERRED EMBODIMENT

The present invention disclosed herein is directed to an injection molded type biometric monitoring ear tag. In the following description, numerous details are set forth in order to provide a thorough understanding of the present invention. It will be appreciated by one skilled in the art that variations of these specific details are possible while still achieving the results of the present invention. In other instance, well-known components are not described in detail in order not to unnecessarily obscure the present invention.

Since the injection molded type biometric monitoring ear tag provided by the present invention can be widely used in various animals to be monitored, so that it will not be described in detail here. Only two embodiments are listed for detailed description, and these embodiments are only used to conveniently and clearly assist in explaining the purpose and effect of the embodiments of the present invention.

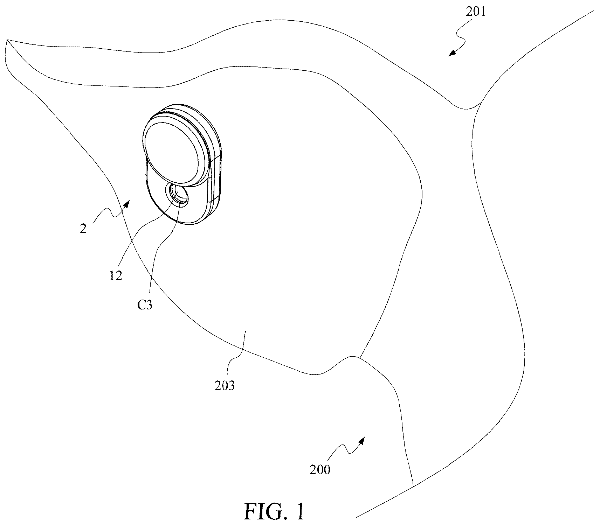

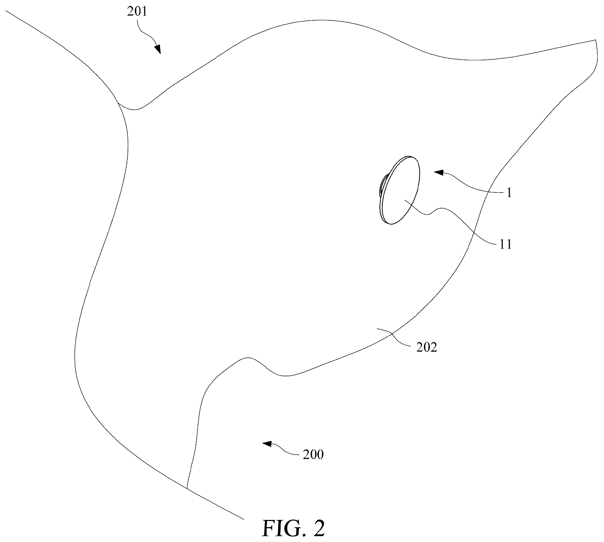

Referring to FIG. 1 and FIG. 2, FIG. 1 is a perspective view showing the female tag of the injection molded type biometric monitoring ear tag provided by the first embodiment of the present invention disposed on the second side of the ear; and FIG. 2 is a perspective view showing the male tag of the injection molded type biometric monitoring ear tag provided by the first embodiment of the present invention disposed on the first side of the ear.

As shown in FIG. 1 and FIG. 2, an injection molded type biometric monitoring ear tag (hereinafter referred to as "ear tag") 100 for attaching to an ear 201 of an animal 200 to be monitored. In the present embodiment, the animal 200 to be monitored is a pig, but it is not limited thereto. In practice, the animal 200 to be monitored can be, for example, an economic animal such as a cow or a sheep.

From a structural perspective, the ear 201 has a first side 202 and a second side 203 opposite to each other. In the present embodiment, the first side 202 is the back of the ear 201 (facing away from the face), and the second side 203 is the front of the ear 201 (facing the face), but it is not limited thereto. In other embodiments, the first side 202 may be the front of the ear 201, and the second side 203 may be the back of the ear 201.

Although the ear tag 100 is set on the ear 201 of the animal 200 to be monitored in the present embodiment, but it is not limited thereto. In practice, it does not exclude that the ear tag 100 may be disposed on other body parts other than the ear 201 by various fixing means (for example, fixing the ear tag 100 on the back of the neck).

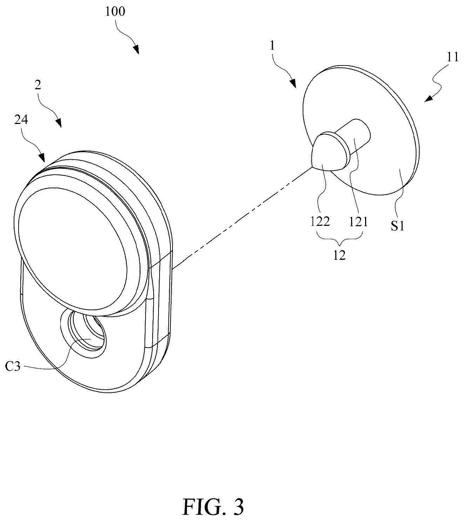

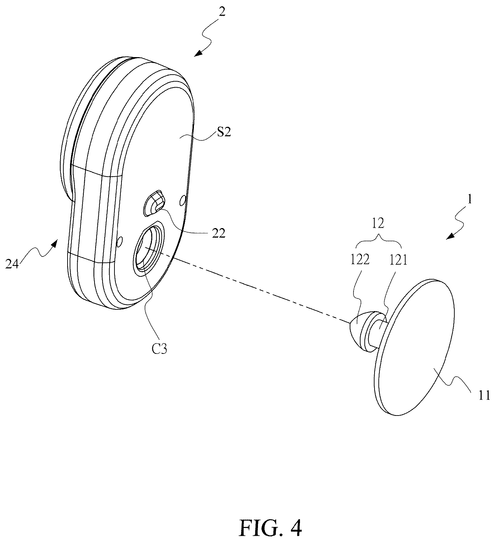

Referring to FIG. 3 and FIG. 4, FIG. 3 is a perspective exploded view of the injection molded type biometric monitoring ear tag provided by the first embodiment of the present invention; and FIG. 4 is a schematic diagram showing a perspective exploded view of the injection molded type biometric monitoring ear tag provided by the first embodiment of the present invention from another perspective. Please also refer to FIG. 1 and FIG. 2 together.

As shown in FIG. 3 and FIG. 4, the ear tag 100 includes a male tag 1 and a female tag 2. The male tag 1 is disposed on the first side 202 and includes a base 11 and a protrusion 12. The base 11 has a base contact surface S1 facing the ear 201. The protrusion 12 extends from the base contact surface S1, for extending from the first side 202 through the ear 201 to the second side 203.

From a structural perspective, the protrusion 12 has an extension segment 121 and a puncture segment 122. The extension segment 121 extends from the base contact surface S1. The puncture segment 122 is facing away from the base contact surface S1 and protrudes from the extension segment 121. The puncture segment 122 is in a cone shape for puncturing the ear 201.

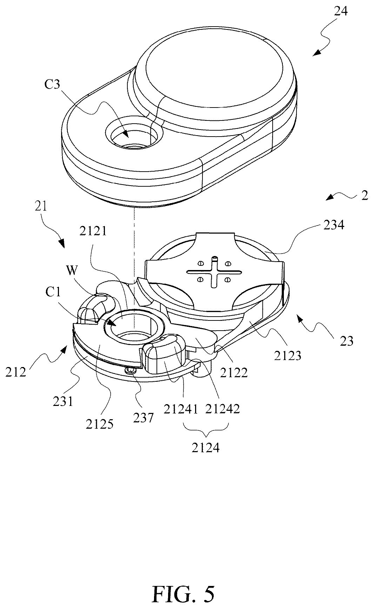

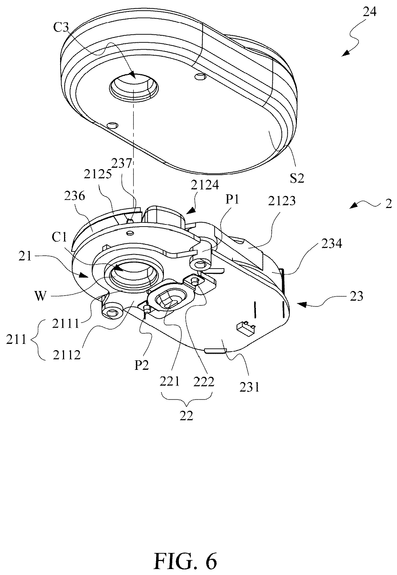

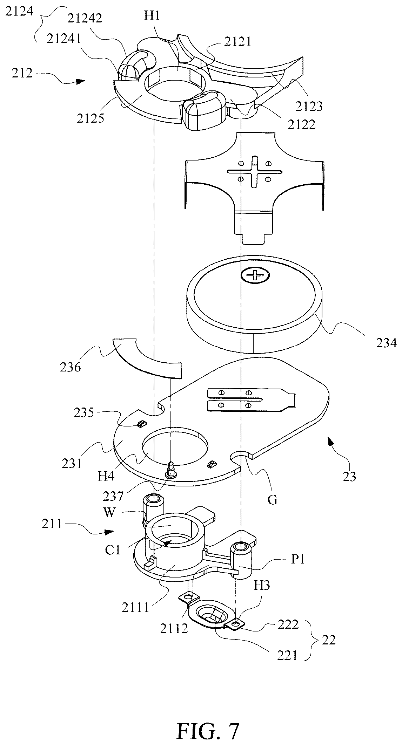

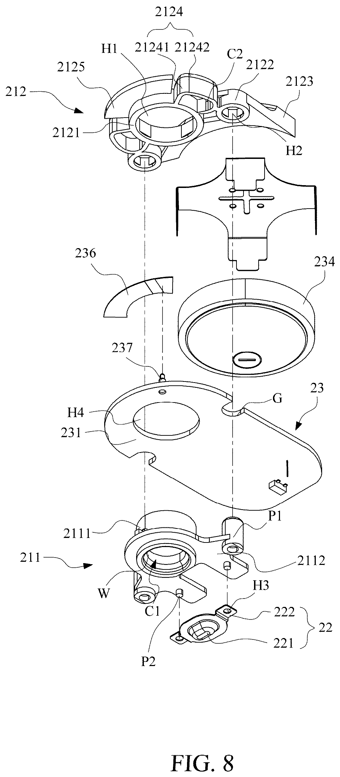

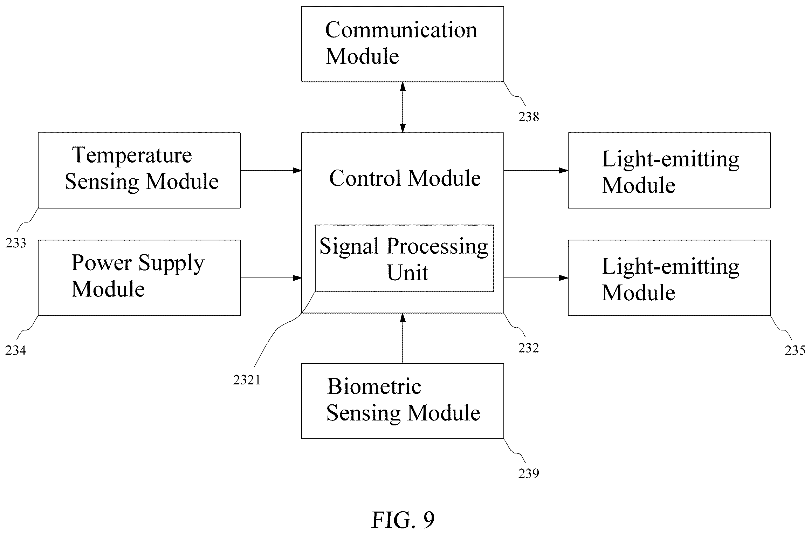

Referring to FIG. 5 to FIG. 9, FIG. 5 is a perspective exploded view of the female tag of the injection molded type biometric monitoring ear tag provided by the first embodiment of the present invention; FIG. 6 is a schematic diagram showing a perspective exploded view of the female tag of the injection molded type biometric monitoring ear tag provided by the first embodiment of the present invention from another perspective; FIG. 7 is a perspective exploded view showing the electronic component of the injection molded type biometric monitoring ear tag provided by the first embodiment of the present invention; FIG. 8 is a perspective exploded view showing the electronic component of the injection molded type biometric monitoring ear tag provided by the first embodiment of the present invention from another perspective; and FIG. 9 is a block diagram showing the implementation system of the injection molded type biometric monitoring ear tag provided by the first embodiment of the present invention. Please also refer to FIG. 1 to FIG. 4 together.

As shown in FIG. 5 to FIG. 9, the female tag 2 is disposed on the second side 203 and includes a fixing bracket 21, at least one heat-conducting element 22, an electronic component 23, and an injection molded shell 24. In the first embodiment, the female tag 2 includes one heat-conducting element 22, but it is not limited thereto. In other embodiments, the female tag 2 may include a plurality of heat-conducting elements 22.

The fixing bracket 21 has a bracket channel C1 surrounded by an inner wall W. From a structural perspective, the fixing bracket 21 includes a lower bracket 211 and an upper bracket 212. The lower bracket 211 includes a lower bracket body 2111 and two lower bracket extensions 2112 (only one is marked). The lower bracket body 2111 has the bracket channel C1. The lower bracket extensions 2112 extend from the lower bracket body 2111 respectively, and each has a snap-fit column P1 (only one is marked) respectively.

The upper bracket 212 includes an upper bracket body 2121 and two upper bracket extensions 2122. The upper bracket body 2121 has a fitting hole H1. The fitting hole H1 is used to fit the lower bracket body 2121. The upper bracket extensions 2122 extend from the upper bracket body 2121 respectively, and each has a snap-fit hole H2 (only one is marked) respectively. The snap-fit holes H2 are used to fit the snap-fit columns P1. The upper bracket 212 is engaged and fixed to the lower bracket 211 when the fitting hole H1 fits the lower bracket body 2111 and the snap-fit holes H2 fit the snap-fit columns P1.

The heat-conducting element 22 is fixed to one side of the fixing bracket 21 facing the ear 201 and used to closely abut the ear 201 to conduct an ear heat energy of the ear 201 when the male tag 1 and the female tag 2 are fixed relative to each other. From the perspective of fixed means, the lower bracket 211 has at least two plug-in columns P2 (only one is marked), and the heat-conducting element 22 includes a heat-conducting protrusion 221 and two fixed ends 222.

In the first embodiment, because only one heat-conducting element 22 is included, the lower bracket 211 only has two plug-in columns P2. In other embodiments, if a plurality of heat-conducting elements 22 are included, the lower bracket 211 needs to be provided with a corresponding number of the plug-in columns P2. The fixed ends 222 extend from the heat-conducting protrusion 221 respectively, and each has a plug-in hole H3 (only one is marked) respectively. The plug-in holes H3 are used to fit the plug-in columns P2, thereby the heat-conducting element 22 is fixed to the lower bracket 211.

The electronic component 23 is fixed to the fixing bracket 21 and coupled to the heat-conducting element 22 to sense an ear temperature value according to the ear heat energy. From the perspective of compositions, the electronic component 23 includes a circuit board 231. The circuit board 231 has a limiting hole H4 and two limiting slots G (only one is marked). The limiting hole H4 is used to fit the lower bracket body 2111, and the limiting slots G are used to accommodate the snap-fit columns P1. The circuit board 231 is restricted and fixed between the lower bracket 211 and the upper bracket 212 when the limiting hole H4 fits the lower bracket body 2111 and the limiting slots G accommodate the snap-fit columns P1. From the perspective of limiting means, the limiting hole H4 can limit the up and down movement of the circuit board 231, and the limiting slots G can limit the rotation of the circuit board 231.

To realize the temperature sending function, the electronic component 23 further includes a control module 232 (marked in FIG. 9), a temperature sensing module 233 (marked in FIG. 9), and a power supply module 234. The control module 232 is disposed on the circuit board 231. The control module 232 may include, for example, a processor or a microcontroller and usually has a signal processing unit 2321, but it is not limited thereto. The signal processing unit 2321 can be used to process various signals, various parameters or various logic judgments from various modules, but it is not limited thereto. The architecture and working principle of the control module 232 and the signal processing unit 2321 are not invented by this invention and will not be described in detail in this embodiment.

The temperature sensing module 233 is disposed on the circuit board 231, electrically connected to the control module 232, and coupled to the heat-conducting element 22 to sense the ear temperature value. The temperature sensing module 233 may include, for example, a thermistor (NTC or PTC), and may calculate the specific ear temperature value based on a change in resistance. The architecture and working principle of the temperature sensing module 233 are prior art and will not be described in detail in this embodiment. In addition, the temperature sensing module 233 can be coupled to the heat-conducting element 22 by, for example, a transmission line (not shown in the figure). The coupling method between the heat-conducting element 22 and the temperature sensing module 233 are not invented by this invention and will not be described in detail in this embodiment.

It should be noted that since the control module 232 and the temperature sensing module 233 can be provided on the circuit board 231 in the form of chips or functional circuits, for example, and there are multiple possible implementation methods. Therefore, in the present invention, only the control module 232 and the temperature sensing module 233 are drawn in the system block diagram (i.e. FIG. 9).

The power supply module 234 is disposed on the circuit board 231 and electrically connected to the control module 232 for supplying the power needed when the electronic component 23 operates. In the present embodiment, the power supply module 234 is a button battery and is disposed on a battery rack (not shown in the figure, the battery rack in this invention includes a positive terminal battery rack and a negative terminal battery rack, and the positive terminal battery rack has four battery springs) on the circuit board 231, but the power supply method in practice is not limited thereto.

In order to prevent the power supply module 234 (button battery) from shifting after being installed on the circuit board 231, the upper bracket 212 further includes a limiting portion 2123. The limiting portion 2123 extends from the upper bracket body 2121 facing the power supply module 234 and is used to abut the power supply module 234 disposed on the circuit board 231, thereby the power supply module 234 is limited. Since the side surface of the button battery is arc-shaped, the limiting portion 2123 has an arc-shaped limiting surface (not shown in the figure) to limit the power supply module 234.

In order to enable livestock farmers to use the ear tag 100 to quickly find the corresponding animal 200 to be monitored or determine the status of the animal 200 to be monitored, the electronic component 23 further includes at least one light-emitting module 235 (only one is marked), and the injection molded shell 24 is a translucent shell. In the present embodiment, two light-emitting modules 235 are used as an example, but the number is not limited thereto in practice.

The light-emitting modules 235 may include, for example, a light-emitting diode (LED), and they are disposed on the circuit board 231 and electrically connected to the control module 232, thereby the light-emitting modules 235 are controlled to generate light transmitting the injection molded shell 24. The light generated by the light-emitting module 235 can be used, for example, to identify the animal 200 to be monitored. For example, a livestock farmer can remotely control the control module 232 to make the light-emitting module 235 emit light, so that the animal 200 to be monitored can be found by visually observing the light source. In addition, it can also be used for determining the status, for example, when the control module 232 determines that the ear temperature value exceeds an abnormal standard value, and the light-emitting module 235 emits light, it can be determined through the visual light source that the animal 200 to be monitored is in an abnormal state.

In order to improve the identification effect, the upper bracket 212 further includes at least one light guide portion 2124 (only one is marked). In the present embodiment, to match the number of the light-emitting module 235, the upper bracket 212 includes two light guide portions 2124. The light guide portions 2124 extend from the upper bracket body 2121 toward the light-emitting modules 235, covers the light-emitting modules 235, and includes a channel portion 21241, and an emitting portion 21242.

The channel portion 21241 has a light guide channel C2, and the emitting portion 21242 is connected to one side of the channel portion 21241 away from the light-emitting modules 235, thereby the light generated by the light-emitting module 235 is guided by the light guide channel C2, and then penetrates the emitting portion 21242 and emits out of the injection molded shell 24. In the present embodiment, the emitting portion 21242 has the shape of a boss (or has special patterns), so that the light can diverge and transmit after penetrating the emitting portion 21242. Compared with concentrated light, the divergent light has a larger visible range and has a better recognition effect. The light guide channel C2 can guide light, thereby preventing the light from overflowing before reaching the emitting portion 21242.

In order to perform remote control, or to implement wireless transmission and reception of parameters, the electronic component 23 further includes an antenna 236, a probe 237, and a communication module 238. The antenna 236 abuts against the abutment portion 2125 of the upper bracket 212. The abutment portion 2125 extends from the upper bracket body 2121. In the present embodiment, the antenna 236 is a thin arc-shaped RF antenna, but it is not limited thereto.

The probe 237 protrudes from the circuit board 231 and is electrically connected to the antenna 236. The communication module 238 is disposed on the circuit board 231 and electrically connected to the control module 232 and the probe 237 respectively, thereby the ear temperature value is controlled to be transmitted via the antenna 236. The communication module 238 may utilize, for example, near field communication technology (NFC), ultra-wideband communication technology (UWB), wireless radio frequency identification technology (RFID), Bluetooth low power consumption technology (BLE), or low power wide area network technology (LPWAN), but it is not limited thereto.

In order to sense biological parameters other than ear temperature value to cope with various situations that may negatively affect the health of the animal 200 to be monitored, the electronic component 23 further includes at least one biometric sensing module 239 (only one is marked). In the present embodiment, one biometric sensing module 239 is used as an example, but the number in practice is not limited thereto. The biometric sensing module 239 is disposed on the circuit board 231, electrically connected to the control module 232, and used to sense at least one biological parameter corresponding to the animal 200 to be monitored.

The biometric sensing module 239 may include, for example, an accelerometer, a motion and position sensor, an ultrasonic sensor, a gyroscope, a humidity sensor, an infrared sensor, a magnetometer, a heart rate sensor, an electrocardiography (ECG), a photoplethysmography sensor (PPG), an acoustic sensor, a vibration sensor, a microphone, a piezoelectric biosensor, a chemical and gas sensor, or a pressure sensor.

When different biometric sensing modules 239 are selected, the corresponding biological parameters will change accordingly. In the present embodiment, all biological parameters are biological parameters, whether related to the organism itself (such as body temperature) or the environment in which the organism is located (such as ambient humidity) are defined as biological parameters.

For example, the ultrasonic sensor can sense the transmission and reception time difference of ultrasonic pulses to measure the distance (the difference between transmission and reception time is the biological parameters corresponding to the ultrasonic sensor); the vibration sensor can sense mechanical vibration, used to monitor the female tag 2 and predict the maintenance; the piezoelectric biosensor can be used for gas sensing, toxicity sensing, etc.; the chemical and gas sensor can sense harmful gases in the environment (such as carbon monoxide, methane, etc.).

In the biometric sensing modules 239 of the present invention, the structure and working principle required for sensing belong to the prior arts and are not technical features of the present invention, so it will not be further described in the present embodiment.

In addition to directly sending the original biological parameters using the communication module 238, the present invention can also select to set at least one parameter processing algorithm in the control module 232. After sensing the biological parameters, the control module 232 will execute the corresponding parameter processing algorithm and only send the processed evaluation results. For example, an acceleration value and angular velocity value sensed by the accelerometer and by the gyroscope can be used to execute an action recognition algorithm, a gait analysis algorithm or a limp detection algorithm to determine whether the walking status of the animal 200 to be monitored is normal and send out the corresponding evaluation results.

Therefore, since the parameters have been initially processed locally, the amount of transmitted parameters can be greatly reduced, thereby saving power consumption and communication bandwidth, thereby realizing edge computing technology.

In addition, each module described in the present invention (including the control module 232, the temperature sensing module 233, the power supply module 234, the light-emitting module 235, the communication module 238, and the biometric sensing modules 239) may be provided on the circuit board 231, for example, in the form of a chip or a functional circuit. The structure and working principle of those modules belong to the prior arts and are not invented by the present invention, so it will not be further described in the present embodiment.

The injection molded shell 24 encapsulates the fixed fixing bracket 21, the heat-conducting element 22, and the electronic component 23 after applying an injection molding process, so that part of the heat-conducting element 22 (part of the heat-conducting protrusion 221) is exposed on one side of the injection molded shell 24 facing the ear 201 (the side of the injection molded shell 24 facing the ear 201 is actually a female tag contact surface S2). Then, it forms a plug-in fixing channel C3 after encapsulating the inner wall W, thereby the protrusion 12 can be plugged and fixed to make the male tag 1 and the female tag 2 relative fixed to each other.

In the present invention, the injection molding process is a low pressure injection molding process, but it is not limited thereto. The process of the low pressure injection molding process is as follows: First, the fixing bracket 21, the heat-conducting element 22 and the electronic component 23, fixed relative to each other, are placed in a low pressure injection mold (not shown in the figure). Then, the heated thermoplastic material (such as hot melt adhesive) is then injected into the mold using a low pressure to form the injection molded shell 24 encapsulating the fixing bracket 21, the heat-conducting element 22 and the electronic component 23 after cooling, thereby forming a one-piece female tag 2.

In other embodiments, as long as the fixing bracket 21, the heat-conducting element 22 and the electronic component 23 that are fixed relative to each other can be sealed and encapsulated after the process is applied, the type of manufacturing process is not limited thereto. In addition, the working principle and corresponding operating equipment of the injection molding process are not technical features claimed by the present invention, and belong to prior arts and will not be further described in the present embodiment.

The means of plug-in fixing includes various methods, for example, after being plugged-in, it is fixed by magnetic action or elastic structure (the corresponding mechanism needs to be provided). For example, the method of magnetic fixation can control the switch state of the magnetic force so that it attracts the protrusion 12 in the fixed state and releases the attraction in the release state; the method of elastic fixation can be switched between the fixed state and the release state through elastic deformation by providing elastic materials (such as rubber, plastic, etc.). The above-mentioned plug-in fixing means are all prior arts, and the specific structure and working principle are not technical features of the present invention, so they will not be further described in the present embodiment.

The one-piece female tag 2 provided by the present invention has been verified by practical experiments that compared with the two-piece female tag 2 in the prior arts (composed of an upper shell and a lower shell), it can effectively reduce the production cost by up to 50%, and shorten the assembly time by nearly 80%, while the yield rate is also increased by about 30%. In addition, the waterproof and dustproof performance of the female tag 2 of the present invention has also been improved from IP65 to IP68.

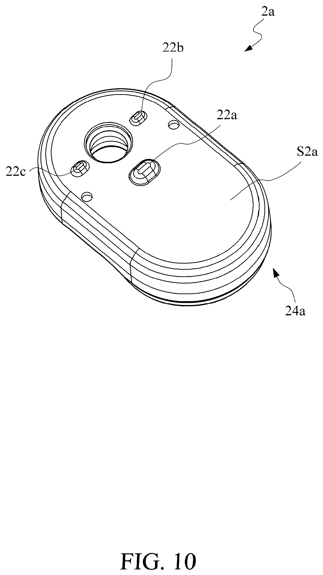

Referring to FIG. 10, FIG. 10 is a perspective exploded view of the injection molded type biometric monitoring ear tag provided by the second embodiment of the present invention. Please also refer to FIG. 1 to FIG. 9 together. As shown in FIG. 10, in the second embodiment, another injection molded type biometric monitoring ear tag (hereinafter referred to as "ear tag") includes a male tag (not shown in the figure) and a female tag 2a in the same manner. The difference from the ear tag 100 of the first embodiment is that the female tag 2a contains three heat-conducting elements 22a, 22b, and 22c. The others are the same or similar to the first embodiment (it also has an injection molded shell 24a, and the injection molded shell 24a also has a female tag contact surface S2a and a plug-in fixing channel C3a). Please refer to the descriptions of the corresponding paragraphs mentioned above.

The heat-conducting elements 22a and 22 are fixed in the same position, and the heat-conducting elements 22b and 22c are respectively fixed on both sides of the plug-in fixing channel C3a. The fixing method of the heat-conducting elements 22b and 22c is similar to or the same as that of the heat-conducting elements 22. A corresponding number of the plug-in columns P2 is required to be provided at the corresponding position of the lower bracket 211. Please refer to the corresponding paragraphs in the first embodiment.

The purpose of using three heat-conducting elements 22a, 22b, 22c is to enable multi-point sensing at three different locations of the ear 201 to improve the representativeness and reliability of temperature sensing. For example, the sensed three ear temperature values can be optimized, analyzed, and calculated to obtain more stable and representative values. In addition, even if one of the heat-conducting elements fails, the other two can still operate continuously, ensuring their backup capabilities and avoiding the impact of temperature sensing functions due to single point failures.

To summarize, the ear tag 100 provided by the present invention is formed after fixing the heat-conducting element 22 and the electronic component 23 on the fixing bracket 21, and is coated with an injection molded shell 24 to form a one-piece female tag 2. Therefore, it not only solves the problems of high costs and time-consuming assembly and increases the feasibility of large-scale production, but also significantly improves waterproof, dustproof and even shockproof performance by eliminating assembly gaps to reduce the risk of damage to internal electronic components.

While the present invention has been particularly shown and described with reference to a preferred embodiment, it will be understood by those skilled in the art that various changes in form and detail may be without departing from the spirit and scope of the present invention.

## Claims

1. An injection molded type biometric monitoring ear tag (100) for attaching to an ear (201) of an animal (200) to be monitored, the ear (201) including a first side (202) and a second side (203), the injection molded type biometric monitoring ear tag (100) comprising:
a male tag (1), disposed on the first side (201), including:
a base (11), having a base contact surface (S1) facing the ear (201); and
a protrusion (12), extending from the base contact surface (S1), for extending from the first side (202) through the ear (201) to the second side (203); and
a female tag (2), disposed on the second side (203), including:
a fixing bracket (21), having a bracket channel (C1) surrounded by an inner wall (W);
at least one heat-conducting element (22, 22a, 22b, 22c), fixed to one side of the fixing bracket (21) facing the ear (201), used to closely abut the ear (201) to conduct an ear heat energy of the ear (201) when the male tag (1) and the female tag (2) are fixed relative to each other;
an electronic component (23), fixed to the fixing bracket (21) and coupled to the at least one heat-conducting element (22, 22a, 22b, 22c) to sense an ear temperature value according to the ear heat energy; and
an injection molded shell (24, 24a), encapsulating the fixed fixing bracket (21), the at least one heat-conducting element (22, 22a, 22b, 22c) and the electronic component (23) after applying an injection molding process, so that part of the at least one heat-conducting element (22, 22a, 22b, 22c) is exposed on one side of the injection molded shell (24, 24a) facing the ear (201), forming a plug-in fixing channel (C3, C3a) after encapsulating the inner wall (W), thereby the protrusion (12) can be plugged and fixed to make the male tag (1) and the female tag (2) relative fixed to each other.

2. The injection molded type biometric monitoring ear tag (100) of claim 1, wherein the fixing bracket (21) comprises:
a lower bracket (211), including:
a lower bracket body (2111), having the bracket channel (C1); and
two lower bracket extensions (2112), extending from the lower bracket body (2111) and having a snap-fit column (P1) respectively; and
an upper bracket (212), including:
an upper bracket body (2121), having a fitting hole (H1), the fitting hole (H1) being used to fit the lower bracket body (2111); and
two upper bracket extensions (2122), extending from the upper bracket body (2121) and having a snap-fit hole (H2) respectively, the snap-fit holes (H2) being used to fit the snap-fit columns (P1);
wherein the upper bracket (212) is engaged and fixed to the lower bracket (211) when the fitting hole (H1) fits the lower bracket body (2111) and the snap-fit holes (H2) fit the snap-fit columns (P1).

3. The injection molded type biometric monitoring ear tag (100) of claim 2, wherein the lower bracket (211) has at least two plug-in columns (P2), the at least one heat-conducting element (22, 22a, 22b, 22c) comprising:
a heat-conducting protrusion (221); and
two fixed ends (222), extending from the heat-conducting protrusion (221) and having a plug-in hole (H3) respectively, the plug-in holes (H3) being used to fit the plug-in columns (P2), thereby the at least one heat-conducting element (22, 22a, 22b, 22c) is fixed to the lower bracket (211).

4. The injection molded type biometric monitoring ear tag (100) of claim 2, wherein the electronic component (23) includes a circuit board (231), the circuit board (231) having a limiting hole (H4) and two limiting slots (G), the limiting hole (H4) being used to fit the lower bracket body (2111), the limiting slots (G) being used to accommodate the snap-fit columns (P1), wherein the circuit board (231) is restricted and fixed between the lower bracket (211) and the upper bracket (212) when the limiting hole (H4) fits the lower bracket body (2111) and the limiting slots (G) accommodate the snap-fit columns (P1).

5. The injection molded type biometric monitoring ear tag (100) of claim 4, wherein the electronic component (23) further comprises:
a control module (232), disposed on the circuit board (231), having a signal processing unit (2321);
a temperature sensing module (233), disposed on the circuit board (231), electrically connected to the control module (232) and coupled to the at least one heat-conducting element (22, 22a, 22b, 22c) to sense the ear temperature value; and
a power supply module (234), disposed on the circuit board (231) and electrically connected to the control module (232).

6. The injection molded type biometric monitoring ear tag (100) of claim 5, wherein the upper bracket (212) further includes a limiting portion (2123), the limiting portion (2123) extending from the upper bracket body (2121) facing the power supply module (234) and being used to abut the power supply module (234) disposed on the circuit board (231), thereby the power supply module (234) is limited.

7. The injection molded type biometric monitoring ear tag (100) of claim 5, wherein the injection molded shell (24, 24a) is a translucent shell and the electronic component (23) further includes at least one light-emitting module (235), the at least one light-emitting module (235) being disposed on the circuit board (231) and electrically connected to the control module (232), thereby the at least one light-emitting module (235) is controlled to generate light transmitting the injection molded shell (24, 24a).

8. The injection molded type biometric monitoring ear tag (100) of claim 7, wherein the upper bracket (212) further includes at least one light guide portion (2124), the at least one light guide portion (2124) extending from the upper bracket body (2121) toward the at least one light-emitting module (235), covering the light-emitting module (235), and including a channel portion (21241) and an emitting portion (21242), the channel portion (21241) having a light guide channel (C2), the emitting portion (21242) being connected to one side of the channel portion (21241) away from the at least one light-emitting module (235), thereby the light generated by the at least one light-emitting module (235) is guided by the light guide channel (C2), and then penetrates the emitting portion (21242) and emits out of the injection molded shell (24, 24a).

9. The injection molded type biometric monitoring ear tag (100) of claim 5, wherein the upper bracket (212) further includes an abutment portion (2125), the abutment portion (2125) extending from the upper bracket body (2121), the electronic component (23) further comprising:
an antenna (236), abutted against the abutment portion (2125);
a probe (237), protruding from the circuit board (231) and electrically connected to the antenna (236); and
a communication module (238), disposed on the circuit board (231) and electrically connected to the control module (232) and the probe (237) respectively, thereby the ear temperature value is controlled to be transmitted via the antenna (236).

10. The injection molded type biometric monitoring ear tag (100) of claim 5, wherein the electronic component (23) further includes at least one biometric sensing module (239), the at least one biometric sensing module (239) being disposed on the circuit board (231) and electrically connected to the control module (232), used to sense at least one biological parameter corresponding to the animal (200) to be monitored, wherein the at least one biometric sensing module (239) is at least one of an accelerometer, a motion and position sensor, an ultrasonic sensor, a gyroscope, a humidity sensor, an infrared sensor, a magnetometer, a heart rate sensor, an electrocardiography (ECG), a photoplethysmography sensor (PPG), an acoustic sensor, a vibration sensor, a microphone, a piezoelectric biosensor, a chemical and gas sensor, and a pressure sensor.
